# FASCICULE DE BREVET EUROPEEN

(11) **EP 2 590 916 B1**
(45) Date de publication et mention de la délivrance du brevet: **12.10.2016**
(21) Numéro de dépôt: 11737991.7
(22) Date de dépôt: 20.06.2011
(51) Int. Cl.: C07C 21/18, C09K 5/04, C10M 105/38, C10M 107/32, C10M 171/00

(54) **COMPOSITION STABLE DE 2,3,3,3-TETRAFLUOROPROPENE**
STABILE 2,3,3,3-TETRAFLUORPROPENZUSAMMENSETZUNG
STABLE 2,3,3,3-TETRAFLUOROPROPENE COMPOSITION

(30) Priorité: 15.07.2010 US 364539 P; 09.07.2010 FR 1055628
(43) Date de publication de la demande: 15.05.2013
(73) Titulaire: Arkema France, 92700 Colombes (FR)
(72) Inventeur: BOUSSAND, Béatrice, F-69110 Sainte Foy Les Lyon (FR)
(74) Mandataire: Dang, Doris
(86) Numéro de dépôt international: PCT/FR2011/051406
(87) Numéro de publication internationale: WO 2012/004487

(56) Documents cités:
- WO-A1-2009/137656
- US-A1- 2006 243 944
- US-A1- 2010 029 997
- US-A1- 2010 119 460
- KNUNYANTS I L ET AL: "Reactions of fluoro olefins. XIII. Catalytic hydrogenation of perfluoro olefins", IZVESTIA AKADEMII NAUK SSSR. SERIA HIMICESKAA, MOSCOW, RU, 1 janvier 1960 (1960-01-01), pages 1312-1318, XP002548816, ISSN: 0002-3353

## Description

La présente invention concerne une composition stable renfermant du 2,3,3,3-tetrafluoropropène, apte à être utilisée en réfrigération et climatisation.

Les problèmes posés par les substances appauvrissant la couche d'ozone atmosphérique ont été traités à Montréal où a été signé le protocole imposant une réduction de la production et de l'utilisation des chlorofluorocarbures (CFC). Ce protocole a fait l'objet d'amendements qui ont imposé l'abandon des CFC et étendu la réglementation à d'autres produits, dont les hydrochlorofluorocarbones (HCFC). L'industrie de la réfrigération et de la climatisation a beaucoup investi dans la substitution de ces fluides frigorigènes et c'est ainsi que les hydrofluorocarbures (HFC) ont été commercialisés.

Dans l'industrie automobile, les systèmes de climatisation des véhicules commercialisés dans de nombreux pays sont passés d'un fluide frigorigène au chlorofluorocarbure (CFC-12) à celui de l'hydrofluorocarbure (1,1,1,2 tetrafluoroéthane : HFC-134a), moins nocif pour la couche d'ozone. Cependant, au regard des objectifs fixés par le protocole de Kyoto, le HFC-134a (GWP = 1430) est considéré comme ayant un pouvoir de réchauffement élevé. La contribution à l'effet de serre d'un fluide est quantifiée par un critère, le GWP (Global Warming Potential) qui résume le pouvoir de réchauffement en prenant une valeur de référence de 1 pour le dioxyde de carbone.

Les hydrofluorooléfines (HFO) ont un pouvoir de réchauffement peu élevé et donc répondent aux objectifs fixés par le protocole de Kyoto. Le document JP 4-110388 divulgue le 2,3,3,3-tetrafluoropropène (HFO-1234yf) comme agent de transfert de chaleur en réfrigération, climatisation et dans les pompes à chaleur.

Outre les bonnes propriétés d'agent de transfert de chaleur, pour qu'un fluide frigorigène soit accepté commercialement il doit notamment présenter une stabilité thermique et une compatibilité avec les lubrifiants. En effet, il est hautement souhaitable que le fluide frigorigène soit compatible avec le lubrifiant utilisé dans le compresseur, présent dans la majorité des systèmes de réfrigération. Cette association fluide frigorigène et lubrifiant est importante pour la mise en oeuvre et l'efficacité du système de réfrigération, notamment le lubrifiant doit être suffisamment soluble dans le fluide frigorigène dans tout l'intervalle de température de fonctionnement.

D'après le document WO 2008/042066, les fluorooléfines étant susceptibles de se dégrader en contact de l'humidité, de l'oxygène ou d'autres composés lors de leur mise en oeuvre comme fluide frigorigène, éventuellement à haute température, il est recommandé de les stabiliser avec au moins une amine.

D'autres stabilisants tels que les dérivés de benzophénone, les lactones et certains composés phosphorés ont également été proposés pour stabiliser les fluorooléfines (WO 2008/027596, WO 2008/027516 et WO 2008/027515).

Par ailleurs, le document EP 2149543 décrit un procédé de purification du 1,1,1,2,3-pentafluoropropane, matière première dans la fabrication du HFO-1234yf, afin d'obtenir un produit ayant une teneur en 1,1,1,2,3-pentafluoropropène (HFO-1225ye) inférieur à 500 ppm et une teneur en trifluoropropyne inférieur à 50 ppm.

La demanderesse a maintenant mis au point une composition du 2,3,3,3-tetrafluoropropène permettant d'améliorer la stabilité thermique lors de sa mise en oeuvre dans des systèmes de réfrigération.

La présente invention a donc pour objet une composition stable (CS) comprenant d'au moins x % en poids de 2,3,3,3-tetrafluoropropène (99,85 ≤ x < 99,98 %), de 0,02 à 0,15 % en poids de composé(s) insaturé(s) (Ia) choisi(s) parmi les isomères de position du 2,3,3,3-tetrafluoropropène tels que le 1,3,3,3-tetrafluoropropène (isomères Z et E), et éventuellement d'au plus 200 ppm de 3,3,3-trifluoropropyne et/ou d'au plus 5 ppm de 1,1,1,2,3-pentafluoropropène (HFO-1225ye) et/ou d'au plus 400 ppm de composés (Ib).

La composition stable selon la présente invention peut comprendre en outre au moins un des composés (Ib) choisis parmi le 1,1,1,2-tetrafluoropropane (HFC-254eb), le 1,1,1,2,3-pentafluoropropane (HFC-245eb), le 1,1,1,2-tetrafluoroethane (HFC-134a), le 1,1,2-trifluoroethane (HFC-143), le 1,1,1,2,3,3-hexafluoropropane, l'hexafluoropropène, le cyclohexafluoropropène et le 1,1,1,3,3-pentafluoropropène (HFO-1225zc).

La totalité des composés (Ib) présents dans la composition selon la présente invention représente au plus 500 ppm.

La composition stable selon l'invention présente l'avantage de pouvoir être obtenue directement par un procédé de fabrication du 2,3,3,3-tetrafluoropropène éventuellement après au moins une étape de séparation.

La présente invention a également pour objet du 2,3,3,3-tetrafluoropropène de pureté supérieure ou égale à 99,8 % et inférieure à 100% en poids et comprenant d'au plus 0,2 % en poids de composés insaturés (Ia), éventuellement d'au plus 500 ppm de 3,3,3-trifluoropropyne et/ou d'au plus 200 ppm de 1,1,1,2,3-pentafluoropropène et/ou d'au plus 500 ppm de composés (Ib).

La présente invention a en outre pour objet du 2,3,3,3-tetrafluoropropène de pureté supérieure à 99,85 % et inférieure à 99,98% en poids et comprenant de 0,02 à 0,15 % en poids de 1,3,3,3-tetrafluoropropène (isomères Z et E), éventuellement d'au plus 200 ppm de 3,3,3-trifluoropropyne et/ou d'au plus 5 ppm de 1,1,1,2,3-pentafluoropropène et/ou d'au plus 500 ppm de composés (Ib).

Le 2,3,3,3-tetrafluoropropène peut-être obtenu à partir de l'hexafluoropropène (HFP) en au moins 4 étapes réactionnelles : - (i) hydrogénation de l'HFP en présence d'un catalyseur d'hydrogénation en phase solide pour donner du 1,1,1,2,3,3-hexafluoropropane ; (ii) déhydrofluoration du 1,1,1,2,3,3-hexafluoropropane obtenu à l'étape (i) en phase liquide à l'aide d'un hydroxyde alcalin ou en phase gazeuse en présence d'un catalyseur de déhydrohalogènation pour donner du 1,1,1,2,3-pentafluoropropène ; (iii) hydrogénation du HFO-1225ye obtenu en (ii) en présence d'un catalyseur d'hydrogénation en phase solide pour donner du 1,1,1,2,3-pentafluoropropane ; (iv) déhydrofluoration du HFC-245eb obtenu à l'étape (iii) en phase liquide à l'aide d'un hydroxyde alcalin ou en phase gazeuse en présence d'un catalyseur de déhydrohalogènation pour donner du 2,3,3,3-tetrafluoropropène.

Le 2,3,3,3-tetrafluoropropène peut-être obtenu à partir de l'hexafluoropropène (HFP) en au moins 2 étapes réactionnelles : - (i) hydrogénation de l'HFP en présence d'un catalyseur d'hydrogénation en phase solide pour donner du 1,1,1,2,3-pentafluoropropane ; (ii) déhydrofluoration du HFC-245eb obtenu à l'étape (i) en phase liquide à l'aide d'un hydroxyde alcalin ou en phase gazeuse en présence d'un catalyseur de déhydrohalogènation pour donner du 2,3,3,3-tetrafluoropropène.

Le 2,3,3,3-tetrafluoropropène selon la présente invention peut être obtenu à partir de l'HFP selon un procédé tel que décrit ci-dessus après purification du HFC-245eb et/ou après purification du 2,3,3,3-tetrafluoropropène.
Ainsi, le HFC-245eb préalablement à l'étape de dèshydrofluoration, est par exemple purifié par distillation à une pression absolue de 6 bar et une température en pied de colonne de 80°C et une température en tête de 50°C avec d'environ 30 plateaux théoriques et un taux de reflux d'environ 37.

Après l'étape finale de déhydrofluoration, le HFO-1234yf est soumis à une double distillation. La première distillation est mise en oeuvre à une pression absolue d'environ 13 bar, une température en pied de colonne d'environ 60°C, une température en tête d'environ 40°C et avec environ 35 plateaux théoriques et un taux de reflux d'environ 500. La deuxième distillation est mise en oeuvre à une pression absolue d'environ 11 bar, une température en pied de colonne d'environ 105°C, une température en tête d'environ 44°C et avec environ 30 plateaux théoriques et un taux de reflux d'environ 4.

Le 2,3,3,3-tetrafluoropropène peut aussi être obtenu à partir du 1,1,1-trifluoro,2-chloropropène par hydrofluoration en phase liquide ou gazeuse en présence d'un catalyseur de fluoration. Le 2,3,3,3-tetrafluoropropène ainsi obtenu peut-être purifié pour donner le 2,3,3,3-tetrafluoropropène selon la présente invention.

Les compositions selon la présente invention sont aptes à être utilisées comme agent de transfert de chaleur dans la climatisation stationnaire ou automobile, réfrigération et pompe à chaleur.

La présente invention a également pour objet les compositions telles que décrites ci-dessus en combinaison avec un lubrifiant.

Comme lubrifiant, on peut citer notamment les esters de polyols (POE), les polyalkylène glycols (PAG), les esters de polyalkylène glycols et les polyvinylethers (PVE).

Les lubrifiants PAG sont sous forme d'homo ou copolymère d'oxyalkylène. Les PAG préférés sont des homopolymères constitués de groupements oxypropylène et ayant une viscosité de 10 à 200 centistokes à 40°C, avantageusement entre 30 et 80 centistokes. Les groupements hydroxy aux extrémités des chaînes d'homo ou copolymère d'oxyalkylène peuvent être plus ou moins remplacés par des groupements -O-CₙH₂ₙ₊₁ avec n = 1 à 10; le groupement avec n= 1 étant préféré.
Les PAG pouvant convenir sont ceux ayant des groupements hydroxy de chaque terminaison ou des groupements -O-CₙH₂ₙ₊₁.

Comme POE, on peut citer notamment les esters d'acides carboxyliques ayant une chaîne carbonée de 2 à 15 atomes, linéaire ou ramifiée et de polyols ayant un squelette de néopentyle comme le néopentyle glycol, le triméthylol propane, le pentaerythritol et le dipentaerythritol ; le pentaerythritol est le polyol préféré. Les esters d'acides carboxyliques de chaîne carbonée de 4 à 9 atomes sont préférés.

Comme acide carboxylique de 4 à 9 atomes de carbone, on peut citer notamment l'acide n-pentanoïque, l'acide n-hexanoïque, l'acide n-heptanoïque, l'acide n-octanoïque, l'acide 2-éthylhexanoïque, l'acide 2,2-diméthylpentanoïque, le 3,5,5-trimethylhexanoïque, l'acide adipique et l'acide succcinique.

Certaines fonctions alcool ne sont pas estérifiées, cependant leur proportion reste faible.

Les huiles POE sélectionnées peuvent contenir entre 0 et 5 % molaire relatif de motifs CH₂-OH par rapport aux motifs -CH₂-O-(C=O)-.
Les lubrifiants POE préférés sont ceux ayant une viscosité de 1 à 1000 centiStokes (cSt) à 40°C, de préférence de 10 à 200 cSt, et avantageusement de 30 à 80 cSt.

### PARTIE EXPERIMENTALE

Les essais de stabilité thermique sont effectués selon la norme ASHRAE 97-2007: "sealed glass tube method to test the chemical stability of materials for use within réfrigérant systems".
Les conditions de test sont les suivantes :
masse de fluide : 2,2 g
masse de lubrifiant : 5 g
température : 200°C
durée : 14 jours

Des coupons d'acier sont introduits dans les tubes.
Le coupon d'acier et le lubrifiant sont introduits dans un tube en verre de 42,2 ml. Le tube est ensuite tiré sous vide puis le fluide F y est ajouté. Le tube est alors soudé pour le fermer et placé dans une étuve à 200°C pendant 14 jours.

En fin de test, différentes analyses sont réalisées :
- la phase gaz est récupérée pour être analysée par chromatographie phase gazeuse : les principales impuretés ont été identifiées par GC/MS (chromatographie phase gazeuse couplée spectrométrie de masse). On peut ainsi regrouper les impuretés venant du fluide F et celles venant du lubrifiant.
- le coupon d'acier est pesé (mesure de la vitesse de corrosion) et observé au microscope.
- le lubrifiant est analysé : couleur (par spectrocolorimétrie, Labomat DR Lange LICO220 Modèle MLG131), humidité (par coulométrie Karl Fischer, Mettler DL37) et indice d'acide (par dosage avec de la potasse méthanolique 0,01N).

Le lubrifiant utilisé dans les tests est une huile PAG commerciale : PAG ND8.
Le fluide utilisé pour ces essais contient essentiellement du HFO-1234yf (au moins 99,9 % en poids) puis on ajoute respectivement 300 ppm de HFO-1243zf ; 500 ppm de HFO-1234ze E et 300 ppm de HFO-1243zf + 500 ppm de HFO-1234ze E au fluide.

| Teneur en | ppm | ppm | ppm | ppm |
|---|---|---|---|---|
| HFO-1234zeE ajoutée | - | - | 500 | 500 |
| HFO-1243zf ajoutée | - | 300 | | 300 |
| Sous-produits dans la phase gaz : | | | | |
| 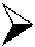 à partir du HFO-1234yf | 600 ppm | 600 ppm | 900 ppm | 900 ppm |
| 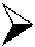 à partir de l'huile | 1,4% | 1,4% | 1,4% | 1,4% |
| Vitesse de corrosion | < 5 µm/an | < 5 µm/an | < 5 µm/an | < 5 µm/an |
| Analyse de l'huile : | | | | |
| 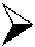 couleur | 10 Gardner | 9 Gardner | 8,5 Gardner | 9 Gardner |
| 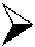 humidité | 300 ppm | 300 ppm | 250 ppm | 300 ppm |
| 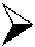 Indice d'acide | 5,2 mg KOH/g | 5,2 mg KOH/g | 4,5 mg KOH/g | 5,2 mg KOH/g |

Les exemples montrent que la présence des composés (Ia) n'est pas nuisible à la stabilité thermique à la fois de la composition du HFO-1234yf que du lubrifiant et dans certains cas l'améliore.

## Revendications

1. Composition stable (CS) comprenant d'au moins x % en poids de 2,3,3,3-tetrafluoropropène (99,85 ≤ x < 99,98 %), de 0,02 à 0,15 % en poids de composé(s) insaturé(s) (Ia choisi(s) parmi les isomères de position du 2,3,3,3-tetrafluoropropène tels que le 1,3,3,3-tetrafluoropropène (isomères Z et E), et éventuellement d'au plus 200 ppm de 3,3,3-trifluoropropyne et/ou d'au plus 5 ppm de 1,1,1,2,3-pentafluoropropène (HFO-1225ye) et/ou d'au plus 400 ppm de composés (Ib).

2. Composition selon la revendication 1 **caractérisée en ce qu'**elle peut comprendre en outre au moins un des composés (Ib) choisis parmi le 1,1,1,2-tetrafluoropropane (HFC-254eb), le 1,1,1,2,3-pentafluoropropane (HFC-245eb), le 1,1,1,2-tetrafluoroethane (HFC-134a), le 1,1,2-trifluoroethane (HFC-143), le 1,1,1,2,3,3-hexafluoropropane, l'hexafluoropropène, le cyclohexafluoropropène et le 1,1,1,3,3-pentafluoropropène (HFO-1225zc).

3. Composition selon la revendication 2 **caractérisée en ce que** le(s) composé(s) (Ib) représente(nt) au plus 500 ppm de la composition.

4. Utilisation d'une composition selon l'une quelconque des revendications précédentes comme agent de transfert de chaleur dans la climatisation stationnaire ou automobile, réfrigération et pompe à chaleur.

5. Utilisation selon la revendication précédente **caractérisée en ce que** la composition est mise en oeuvre avec un lubrifiant.

6. Utilisation selon la revendication précédente **caractérisée en ce que** le lubrifiant est choisi parmi les esters de polyols (POE), les polyalkylène glycols (PAG), les esters de polyalkylène glycols et les polyvinylethers (PVE).

7. 2,3,3,3-tetrafluoropropène de pureté supérieure à 99,85 % et inférieure à 99,98% en poids et comprenant de 0,02 à 0,15 % en poids de 1,3,3,3-tetrafluoropropène (isomères Z et E), éventuellement d'au plus 200 ppm de 3,3,3-trifluoropropyne et/ou d'au plus 5 ppm de 1,1,1,2,3-pentafluoropropène et/ou d'au plus 500 ppm de composés (Ib).

## Patentansprüche

1. Stabile Zusammensetzung (SZ), umfassend mindestens x Gew.-% 2,3,3,3-Tetrafluorpropen (99,85 ≤ x < 99,98%), 0,02 bis 0,15 Gew.-% ungesättigte Verbindung(en) (Ia), die aus den Stellungsisomeren von 2,3,3,3-Tetrafluorpropen, wie 1,3,3,3-Tetrafluorpropen (Z- und E-Isomer), ausgewählt sind, und gegebenenfalls höchstens 200 ppm 3,3,3-Trifluorpropin und/oder höchstens 5 ppm 1,1,1,2,3-Pentafluorpropen (HFO-1225ye) und/oder höchstens 400 ppm Verbindungen (Ib).

2. Zusammensetzung nach Anspruch 1, **dadurch gekennzeichnet, dass** sie außerdem mindestens eine der Verbindungen (Ib), die aus 1,1,1,2-Tetrafluorpropan (H-FKW 254eb), 1,1,1,2,3-Pentafluorpropan (H-FKW 245eb), 1,1,1,2-Tetrafluorethan (H-FKW 134a), 1,1,2-Trifluorethan (H-FKW 143), 1,1,1,2,3,3-Hexafluorpropan, Hexafluorpropen, Cyclohexafluorpropen und 1,1,1,3,3-Pentafluorpropen (HFO-1225zc) ausgewählt sind, umfassen kann.

3. Zusammensetzung nach Anspruch 2, **dadurch gekennzeichnet, dass** die Verbindung bzw. die Verbindungen (Ib) höchstens 500 ppm der Zusammensetzung ausmacht bzw. ausmachen.

4. Verwendung einer Zusammensetzung nach einem der vorhergehenden Ansprüche als Wärmeübertragungsmittel bei der ortsfesten Klimatisierung oder Automobil-Klimatisierung, bei der Kälteerzeugung und in Wärmepumpen.

5. Verwendung nach dem vorhergehenden Anspruch, **dadurch gekennzeichnet, dass** die Zusammensetzung mit einem Schmiermittel eingesetzt wird.

6. Verwendung nach dem vorhergehenden Anspruch, **dadurch gekennzeichnet, dass** das Schmiermittel aus Polyolestern (POE), Polyalkylenglykolen (PAG), Polyalkylenglykolestern und Polyvinylethern (PVE) ausgewählt ist.

7. 2,3,3,3-Tetrafluorpropen, das eine Reinheit von mehr als 99,85 Gew.-% aufweist und 0,02 bis 0,15 Gew.-% 1,3,3,3-Tetrafluorpropen (Z- und E-Isomer), gegebenenfalls höchstens 200 ppm 3,3,3-Trifluorpropin und/oder höchstens 5 ppm 1,1,1,2,3-Pentafluorpropen und/oder höchstens 500 ppm Verbindungen (Ib) umfasst.

## Claims

1. Stable composition (SC) comprising at least x% by weight of 2,3,3,3-tetrafluoropropene (99.85 ≤ x < 99.98%), from 0.02 to 0.15% by weight of unsaturated compound(s) (Ia) chosen from the positional isomers of 2,3,3,3-tetrafluoropropene, such as 1,3,3,3-tetrafluoropropene (Z and E isomers), and optionally at most 200 ppm of 3,3,3-trifluoropropyne and/or at most 5 ppm of 1,1,1,2,3-pentafluoropropene (HFO-1225ye) and/or at most 400 ppm of compounds (Ib).

2. Composition according to Claim 1, **characterized in that** it can additionally comprise at least one of the compounds (Ib) chosen from 1,1,1,2-tetrafluoropropane (HFC-254eb), 1,1,1,2,3-pentafluoropropane (HFC-245eb), 1,1,1,2-tetrafluoroethane (HFC-134a), 1,1,2-trifluoroethane (HFC-143), 1,1,1,2,3,3-hexafluoropropane, hexafluoropropene, cyclohexafluoropropene and 1,1,1,3,3-pentafluoropropene (HFO-1225zc).

3. Composition according to Claim 2, **characterized in that** the compound(s) (Ib) represent(s) at most 500 ppm of the composition.

4. Use of a composition according to any one of the preceding claims as heat transfer agent in stationary or motor-vehicle air conditioning, refrigeration and heat pumps.

5. Use according to the preceding claim, **characterized in that** the composition is employed with a lubricant.

6. Use according to the preceding claim, **characterized in that** the lubricant is chosen from polyol esters (POEs), polyalkylene glycols (PAGs), polyalkylene glycol esters and polyvinyl ethers (PVEs).

7. 2,3,3,3-Tetrafluoropropene which has a purity of greater than 99.85% by weight and less than 99.98% by weight and which comprises from 0.02 to 0.15% by weight of 1,3,3,3-tetrafluoropropene (Z and E isomers), optionally at most 200 ppm of 3,3,3-trifluoropropyne and/or at most 5 ppm of 1,1,1,2,3-pentafluoropropene and/or at most 500 ppm of compounds (Ib).
